(19) Europäisches Patentamt
European Patent Office
Office européen des brevets

(11) EP 0 689 549 B1

(12) # EUROPÄISCHE PATENTSCHRIFT

(45) Veröffentlichungstag und Bekanntmachung des Hinweises auf die Patenterteilung:
**17.06.1998 Patentblatt 1998/25**

(21) Anmeldenummer: **94911129.8**

(22) Anmeldetag: **09.03.1994**

(51) Int Cl.$^6$: **C07K 5/06**, C07K 5/08, C07C 275/40, C07C 275/42, C07D 263/32, A61K 38/04

(86) Internationale Anmeldenummer:
**PCT/EP94/00713**

(87) Internationale Veröffentlichungsnummer:
**WO 94/22907 (13.10.1994 Gazette 1994/23)**

(54) **NEUE HARNSTOFFDERIVATE, IHRE HERSTELLUNG UND VERWENDUNG**

NEW UREA DERIVATES, THEIR PREPARATION AND THEIR USE

NOUVEAUX DERIVES D'UREE, LEUR PREPARATION ET LEUR UTILISATION

(84) Benannte Vertragsstaaten:
**AT BE CH DE DK ES FR GB GR IE IT LI LU MC NL PT SE**

(30) Priorität: **26.03.1993 DE 4309867**

(43) Veröffentlichungstag der Anmeldung:
**03.01.1996 Patentblatt 1996/01**

(73) Patentinhaber: **HOECHST AKTIENGESELLSCHAFT**
**65926 Frankfurt am Main (DE)**

(72) Erfinder:
- **KLINGLER, Otmar**
  **D-63110 Rodgau (DE)**
- **JABLONKA, Bernd**
  **D-65812 Bad Soden (DE)**
- **JUST, Melitta**
  **D-63225 Langen (DE)**
- **ZOLLER, Gerhard**
  **D-61137 Schöneck (DE)**
- **BREIPOHL, Gerhard**
  **D-60529 Frankfurt am Main (DE)**
- **KNOLLE, Jochen**
  **D-65830 Kriftel (DE)**
- **KÖNIG, Wolfgang**
  **D-94375 Stallwang (DE)**
- **STILZ, Hans-Ulrich**
  **D-60529 Frankfurt am Main (DE)**

(56) Entgegenhaltungen:
**EP-A- 0 512 829**
**EP-A- 0 513 675**
**WO-A-92/13552**
**DE-A- 4 126 277**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte Harnstoffe und Thioharnstoffe, ihre Herstellung und ihre Verwendung als Heilmittel, insbesondere als Hemmstoffe der Blutplättchenaggregation.

In der EP-A 449 079 und der EP-A 530 505 sind Hydantoinderivate beschrieben, die thrombozytenaggregationshemmende Wirkungen aufweisen. Strukturell verwandte Harnstoffderivate werden in der WO-A 92 13552 und der EP-A 512 829 erwähnt. Weitere Untersuchungen zeigten, daß auch die Harnstoffderivate der vorliegenden Erfindung starke Hemmstoffe der Blutplättchenaggregation sind.

Gegenstand der vorliegenden Erfindung sind Harnstoffderivate der allgemeinen Formel I,

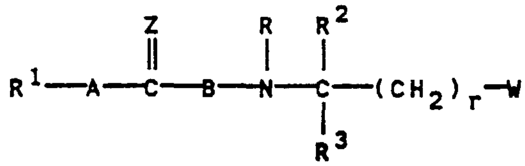

worin

r für die Zahlen 0 oder 1 steht;

Z Sauerstoff oder Schwefel bedeutet;

W $-COW^1$ oder Tetrazolyl bedeutet;

$W^1$ Hydroxy, $(C_1-C_4)$-Alkoxy, Benzyloxy, Amino oder Mono- oder Di-($(C_1-C_8)$-alkyl)-amino bedeutet;

A

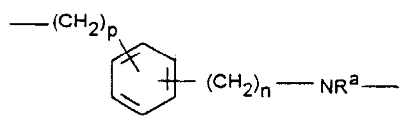

bedeutet, worin p für die Zahl 0 steht und n für die Zahlen 0 oder 1 steht;

B $-NR^b-(CH_2)_m-CO-$ bedeutet, worin m für die Zahlen 1 oder 2 steht;

$R^a$ und $R^b$ unabhängig voneinander Wasserstoff, Hydroxy, $(C_1-C_{18})$-Alkyl, $(C_6-C_{14})$-Aryl, $(C_6-C_{14})$-Aryl-$(C_1-C_8)$-alkyl, $(C_1-C_{28})$-Alkoxy, $(C_6-C_{14})$-Aryloxy, $(C_6-C_{14})$-Aryl-$(C_1-C_8)$-alkoxy bedeuten;

R Wasserstoff oder $(C_1-C_6)$-Alkyl bedeutet;

$R^1$ -NH-X oder $-C(=NX)-NH_2$ bedeutet;

X Wasserstoff, $(C_1-C_6)$-Alkylcarbonyl, $(C_1-C_6)$-Alkoxycarbonyl, $(C_1-C_{18})$-Alkylcarbonyloxy-$(C_1-C_6)$-alkoxycarbonyl, $(C_6-C_{14})$-Aryl-$(C_1-C_6)$-alkoxycarbonyl, Hydroxy oder einen Rest der Formel II

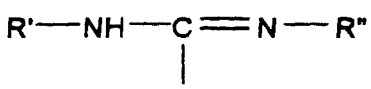

(II)

bedeutet, wobei R' und R" unabhängig voneinander für Wasserstoff, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryl-$(C_1-C_6)$-alkoxycarbonyl oder Hydroxy stehen;

$R^2$ Wasserstoff oder Phenyl bedeutet;

$R^3$ Wasserstoff oder $-CO-NH-R^4$ bedeutet, wobei $-NH-R^4$ für den Rest einer $\alpha$-Aminosäure, deren $\omega$-Amino-$(C_2-C_8)$-alkylamid oder deren $(C_1-C_8)$-Alkyl- oder Benzylester steht, oder wobei $R^4$ Methyl bedeutet, das durch eine Aminosäureseitenkette und durch einen Rest aus der Reihe $-SO_2-OH$, $-SO_2-NHR^9$, Tetrazolyl substituiert ist;

$R^9$ Wasserstoff, Aminocarbonyl, $(C_1-C_{18})$-Alkylaminocarbonyl, $(C_3-C_8)$-Cycloalkylaminocarbonyl, $(C_1-C_{18})$-Alkyl oder $(C_3-C_8)$-Cycloalkyl bedeutet;

sowie deren physiologisch verträgliche Salze.

Cycloalkylreste sind insbesondere Cyclopropyl, Cyclobutyl, Cyclopentyl, Cyclohexyl, Cycloheptyl und Cyclooctyl, die aber auch durch beispielsweise $(C_1-C_4)$-Alkyl substituiert sein können. Beispiele für substituierte Cycloalkylreste

sind 4-Methylcyclohexyl und 2,3-Dimethylcyclopentyl.

Alkylreste können geradkettig oder verzweigt sein. Dies gilt auch, wenn sie Substituenten tragen oder als Substituenten anderer Reste auftreten. Beispiele für geeignete ($C_1$-$C_{18}$)-Alkylreste sind: Methyl, Ethyl, Propyl, Butyl, Pentyl, Hexyl, Heptyl, Octyl, Decyl, Undecyl, Dodecyl, Tridecyl, Pentadecyl, Hexadecyl, Heptadecyl, Octadecyl, Isopropyl, Isobutyl, Isopentyl, Neopentyl, Isohexyl, 3-Methylpentyl, 2,3,5-Trimethylhexyl, sec.-Butyl, tert.-Butyl, tert.-Pentyl. Bevorzugte Alkylreste sind Methyl, Ethyl, Propyl, Isopropyl, Butyl, Isobutyl, sec.-Butyl und tert.-Butyl.

($C_6$-$C_{14}$)-Arylgruppen sind beispielsweise Phenyl, Naphthyl, Biphenylyl oder Fluorenyl, wobei Phenyl und Naphthyl bevorzugt sind. Entsprechendes gilt für Reste wie Aralkyl oder Arylcarbonyl. Aralkylreste sind insbesondere Benzyl sowie 1- und 2-Naphthylmethyl, die auch substituiert sein können. Substituierte Aralkylreste sind beispielsweise Halobenzyl oder ($C_1$-$C_4$)-Alkoxybenzyl.

Ist Phenyl zweifach substituiert, können die Substituenten in 1,2-, 1,3- oder 1,4-Position zueinander stehen. Die 1,3- sowie die 1,4-Position sind bevorzugt.

Natürliche und unnatürliche Aminosäuren können, falls chiral, in der D- oder L-Form vorliegen. Bevorzugt sind α-Aminosäuren. Beispielsweise seien genannt (vgl. Houben-Weyl, Methoden der organischen Chemie, Band XV/1 und 2, Stuttgart, 1974):

**Aad, Abu γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp,**

**Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.**

Unter Aminosäureseitenketten werden Seitenketten von natürlichen oder unnatürlichen Aminosäuren verstanden.

Azaaminosäuren sind natürliche oder unnatürliche Aminosäuren, in denen der Zentralbaustein

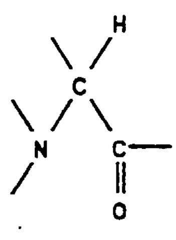

durch

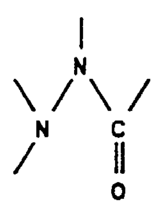

ersetzt ist.

Als Rest einer Iminosäure kommen insbesondere Reste von Heterocyclen aus der folgenden Gruppe in Betracht:

Pyrrolidin-2-carbonsäure; Piperidin-2-carbonsäure; Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1$^{6,9}$]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxypyrrolidin-2-carbonsäure; die alle gegebenenfalls substituiert sein können (siehe folgende Formeln):

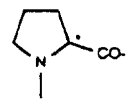 ; 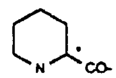 ;

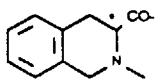 . 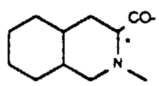 ; 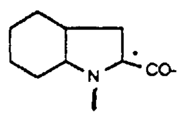 ; 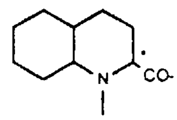 ;

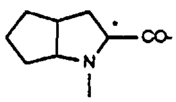 ; 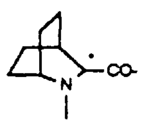 ; 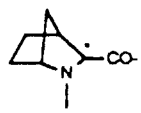 ; 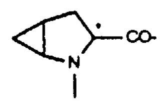 ;

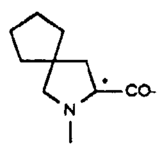 ; 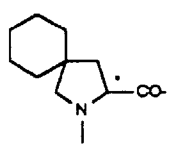 ; 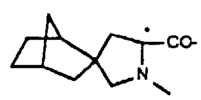 ;

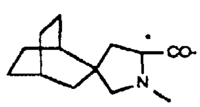 ; 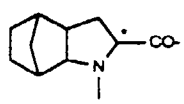 ; 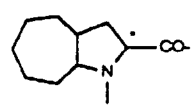 ;

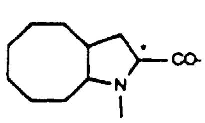 ; 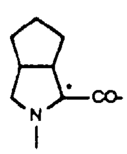 ; 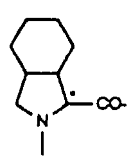 ;

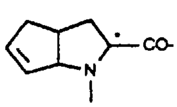 ; 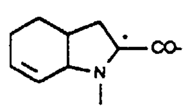 ; 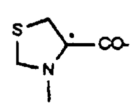 ;

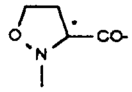 ; 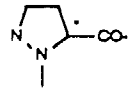 ; 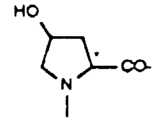 ;

Die oben genannten Resten zugrundeliegenden Heterocyclen sind beispielsweise bekannt aus US-A 4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren und Dipeptide auch als Ester bzw. Amide vorliegen, wie z. B. Methylester, Ethylester, Isopropylester, Isobutylester, tert.-Butylester, Benzylester, Ethylamid, Semicarbazid oder ω-Amino-($C_2$-$C_8$)-alkylamid.

Funktionelle Gruppen der Aminosäuren, Iminosäuren und Dipeptide können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z ($NO_2$), Z($Hal_n$), Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin, Ethanolamin oder Tris-(2-hydroxy-ethyl)-amin.

Verbindungen der allgemeinen Formel I, welche basische Gruppen, z. B. eine Aminogruppe, eine Amidinogruppe oder eine Guanidinogruppe enthalten, bilden mit anorganischen Säuren, wie z. B. Salzsäure, Schwefelsäure oder Phosphorsäure und mit organischen Carbon- oder Sulfonsäuren, wie z. B. Essigsäure, Citronensäure, Benzoesäure, Maleinsäure, Fumarsäure, Weinsäure, Methansulfonsäure oder p-Toluolsulfonsäure Salze.

Die erfindungsgemäßen Verbindungen der allgemeinen Formel I können optisch aktive Kohlenstoffatome enthalten und somit in Form reiner Enantiomerer oder in Form von Enantiomerengemischen vorliegen. Sowohl reine Enantiomere und Enantiomerengemische als auch Diastereomere und Diastereomerengemische sind Gegenstand der vorliegenden Erfindung.

Die erfindungsgemäßen Verbindungen der allgemeinen Formel I können darüber hinaus bewegliche Wasserstoffatome enthalten, also in verschiedenen tautomeren Formen vorliegen. Auch diese Tautomeren sind Gegenstand der vorliegenden Erfindung.

Bevorzugt sind Verbindungen der allgemeinen Formel I, worin

r die Zahl 1 bedeutet;
**W** -$COW^1$ bedeutet;
**$W^1$** Hydroxy, ($C_1$-$C_4$)-Alkoxy, insbesondere Methoxy, Ethoxy, 2-Propyloxy, Isobutyloxy oder tert.-Butyloxy, oder Benzyloxy bedeutet;
**A** -$(CH_2)_k$-$NR^a$-, worin k für die Zahlen 1 oder 2 steht, oder

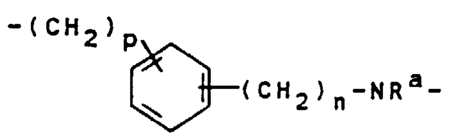 ,

worin n für die Zahlen 0 oder 1 und p für die Zahl 0 steht, bedeutet;
**B** -$NR^b$-$(CH_2)_m$-CO-, worin m für die Zahlen 1 oder 2 steht, oder -$NR^b$-$CHR^s$-CO-, worin **$R^s$** für die Seitenkette der Aminosäuren Alanin, Valin, Phenylalanin, Tyrosin, Leucin, Isoleucin, Tryptophan, Lysin, Histidin, Asparagin, Glutamin oder Phenylglycin steht, oder

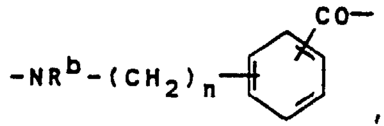
,

worin n für die Zahlen 0 oder 1 steht, oder

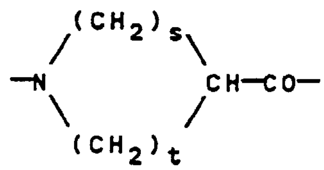
,

worin s und t unabhängig voneinander für eine ganze Zahl von 0 bis 4 stehen können, die Summe von s und t aber die Zahl 3 oder die Zahl 4 ergeben muß, bedeutet, wobei aber, wenn A für $-(CH_2)_k-NR^a-$ steht und darin k für 2 steht, dann nicht zugleich B $-NR^b-(CH_2)_m-CO-$ bedeuten kann;

**R** Wasserstoff bedeutet;

**X** Wasserstoff, ($C_1$-$C_6$)-Alkoxycarbonyl, ($C_1$-$C_6$)-Alkylcarbonyl, ($C_1$-$C_{18}$)-Akylcarbonyloxy-($C_1$-$C_6$)-alkoxycarbonyl, ($C_6$-$C_{14}$)-Aryl-($C_1$-$C_6$)-alkoxycarbonyl oder einen Rest der Formel

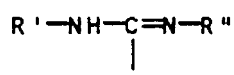

bedeutet, worin R' und R" unabhängig voneinander für Wasserstoff, Trifluorethyl, ($C_1$-$C_6$)-Alkylcarbonyl, ($C_1$-$C_6$)-Alkoxycarbonyl, ($C_1$-$C_{18}$)-Akylcarbonyloxy-($C_1$-$C_6$)-alkoxycarbonyl oder ($C_6$-$C_{14}$)-Aryl-($C_1$-$C_6$)-alkoxycarbonyl stehen;

**$R^2$** Wasserstoff bedeutet;

**$R^3$** $-CO-NH-R^4$ bedeutet, wobei $-NH-R^4$ für den Rest einer α-Aminosäure, deren ω-Amino-($C_2$-$C_8$)-alkylamid oder deren ($C_1$-$C_8$)-Alkyl- oder Benzylester steht, oder wobei $R^4$ Methyl bedeutet, das durch eine Aminosäureseitenkette und durch einen Rest aus der Reihe $-SO_2-OH$, $-SO_2-NHR^9$, Tetrazolyl substituiert ist.

Für $-NH-R^4$ stehende Reste von α-Aminosäuren sind dabei besonders bevorzugt der Valin-, Lysin-, Phenylalanin-, Phenylglycin- oder 4-Chlorphenylglycin-Rest. Steht $-NH-R^4$ dabei für einen Ester einer dieser α-Aminosäuren, so ist der Methyl-, Ethyl-, Isopropyl-, Isobutyl-, tert.-Butylester oder Benzylester bevorzugt.

Verbindungen der Formel I können hergestellt werden durch Fragmentkondensation einer Verbindung der allgemeinen Formel III

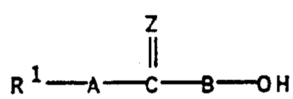
(III)

mit einer Verbindung der allgemeinen Formel IV

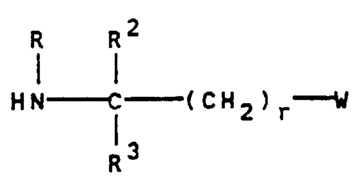
(IV)

wobei die Reste A, B, W, Z, R, $R^1$, $R^2$ und $R^3$ sowie r wie oben angegeben definiert sind.

Die Ausgangsverbindungen der allgemeinen Formel IV werden in der Regel vom C-terminalen Ende her stufenweise aufgebaut. Zur Kondensation der Verbindungen der allgemeinen Formel III mit denen der allgemeinen Formel IV verwendet man vorteilhafterweise die an sich bekannten Kupplungsmethoden der Peptidchemie (siehe z. B. Houben-Weyl, Methoden der Organischen Chemie, Band 15/1 und 15/2, Stuttgart, 1974). Dazu ist es in der Regel nötig, daß in $R^1$, $R^2$, $R^3$ und W enthaltene Aminogruppen durch reversible Schutzgruppen während der Kondensation geschützt werden. Gleiches gilt für die Carboxylgruppen der Verbindungen der Formel IV, die bevorzugt als ($C_1$-$C_6$)-Alkyl, Benzyl- oder tert.-Butylester vorliegen. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können $NO_2$-Gruppen (Guanidinoschutz), Benzyloxycarbonylgruppen und Benzylester abhydriert werden. Die Schutzgruppen vom tert.-Butyltyp werden sauer gespalten, während der 9-Fluorenylmethyloxycarbonylrest durch sekundäre Amine entfernt wird.

Die Ausgangsverbindungen der allgemeinen Formel III können wie folgt erhalten werden:

Aminderivate der allgemeinen Formel V bzw. Aminderivate der allgemeinen Formel VI, in denen, wie beispielhaft gezeigt, die Carbonsäuregruppe als Methylester vorliegen kann, wobei A, B und $R^1$

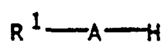
(V)

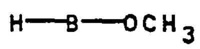
(VI)

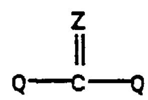
(VII)

die oben angegebenen Bedeutungen haben, können mit reaktiven Kohlensäurederivaten der allgemeinen Formel VII, worin Z für Sauerstoff oder Schwefel steht und die beiden Reste Q gleiche oder verschiedene Abgangsgruppen darstellen, zu den Verbindungen der allgemeinen Formel VIII bzw. den Verbindungen der allgemeinen Formel IX

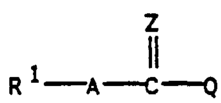
(VIII)

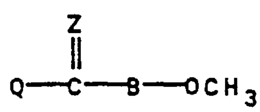
(IX)

umgesetzt werden. Geeignete Abgangsgruppen Q sind beispielsweise Halogenide, insbesondere Chlorid, ($C_1$-$C_4$)-Alkoxy, beispielsweise Methoxy, Ethoxy oder Isobutoxy, ($C_1$-$C_4$)-Alkylthio, beispielsweise Methylthio oder Ethylthio, unsubstituiertes ($C_6$-$C_{14}$)-Aryloxy, insbesondere Phenoxy, ein- oder mehrfach substituiertes ($C_6$-$C_{14}$)-Aryloxy, insbesondere substituiertes Phenoxy, beispielsweise 4-Nitrophenoxy, 4-Chlorphenoxy oder 2,4,5-Trichlorphenoxy, oder Di- und Triazolylreste, beispielsweise Imidazolyl oder Triazolyl. Beispiele für reaktive Kohlensäurederivate sind somit Phosgen, das auch in Form von Di- oder Triphosgen eingesetzt werden kann, Thiophosgen, Chlorameisensäurealkyl- und -arylester, Dialkyl- und Diarylcarbonate, in denen sich die beiden Reste auch unterscheiden können, Thiocarbonate, N,N'-Carbonyldiimidazol, N,N'-Thiocarbonyldiimidazol, 1,1'-Carbonyldi-1,2,4-triazol oder 1,1'-Carbonyl-dibenzotriazol. Die Kondensation der Verbindungen der allgemeinen Formel VIII mit den Verbindungen der allgemeinen Formel VI bzw. die Kondensation der Verbindungen der allgemeinen Formel IX mit den Verbindungen der allgemeinen Formel V liefert nach Verseifung die Verbindungen der allgemeinen Formel III (s.z.B. Houben-Weyl, Methoden der Organischen Chemie, Bände VIII und E 4, Stuttgart 1952 bzw. 1983).

Wenn einer der beiden Reste $R^a$ und $R^b$ für Wasserstoff steht oder beide für Wasserstoff stehen, können die Ausgangsverbindungen der allgemeinen Formel III auch unter Verwendung von Heterokumulenen hergestellt werden:

Die Reaktion von Aminocarbonsäureestern beispielsweise der allgemeinen Formel VI

$$H\text{-}B\text{-}OCH_3 \qquad (VI),$$

worin B die oben angegebenen Bedeutungen hat, mit einem Isocyanat oder einem Isothiocyanat der allgemeinen Formel X

$$R^1\text{-}Y^a\text{-}N{=}C{=}Z \qquad (X),$$

worin $R^1$ und Z die oben angegebenen Bedeutungen haben und $Y^a$ für $-(CH_2)_k-$ oder

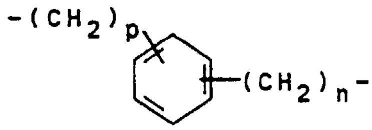

steht, worin k, n und p die oben angegebenen Bedeutungen haben, führt zu den Verbindungen der allgemeinen Formel IIIa

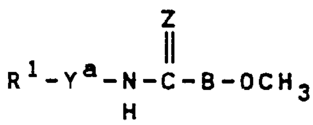

(IIIa)

bzw. - nach Verseifung der Estergruppe - zu den entsprechenden Carbonsäuren.

Die Reaktion von Aminderivaten der allgemeinen Formel V

$$R^1\text{-}A\text{-}H \qquad (V)$$

worin $R^1$ und A die oben angegebenen Bedeutungen haben, mit einem Isocyanato- oder Isothiocyanatocarbonsäureester der allgemeinen Formel XI

$$Z{=}C{=}N\text{-}Y^b\text{-}OCH_3 \qquad (XI)$$

worin Z für Sauerstoff oder Schwefel steht und $Y^b$ für $-(CH_2)_m-CO-$ oder $-CHR^s-CO-$ oder

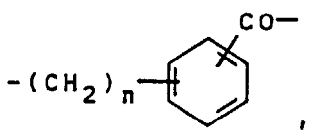

,

steht, worin $R^s$ und m und n die oben angegebenen Bedeutungen haben, führt zu den Verbindungen der allgemeinen Formel IIIb

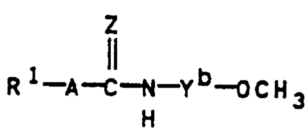

(IIIb)

bzw. - nach Verseifung der Estergruppe - zu den entsprechenden Carbonsäuren.

Bei allen Reaktionsschritten müssen gegebenenfalls freie funktionelle Gruppen durch geeignete reversible Schutzgruppen blockiert werden, die später in geeigneter Weise wieder abgespalten werden.

Für die Guanylierung und Nitroguanylierung der Aminoverbindungen können folgende Reagentien verwendet werden:

1. O-Methylisoharnstoff
(S. Weiss und H. Krommer, Chemiker Zeitung 98 (1974) 617 - 618),
2. S-Methylisothioharnstoff
R.F. Borne, M.L. Forrester und I.W. Waters, J. Med. Chem. 20 (1977) 771 - 776),
3. Nitro-S-methylisothioharnstoff
(L.S. Hafner und R.E. Evans, J. Org. Chem. 24 (1959) 1157),
4. Formamidinsulfonsäure
(K. Kim, Y.-T. Lin und H.S. Mosher, Tetrahedron Lett. 29 (1988) 3183 - 3186),
5. 3,5-Dimethyl-1-pyrazolyl-formamidinium-nitrat
(F.L. Scott, D.G. O'Donovan und J. Reilly, J. Amer. Chem. Soc. 75 (1953) 4053 - 4054),
6. N,N'-Di-tert.-butyloxycarbonyl-S-methyl-isothioharnstoff
(R.J. Bergeron und J.S. McManis, J. Org. Chem. 52 (1987) 1700 - 1703),
7. N-Alkoxycarbonyl-, N,N'-Dialkoxycarbonyl-, N-Alkylcarbonyl- und N,N'-Dialkylcarbonyl-S-methyl-isothioharnstoff (H. Wollweber, H. Kölling, E. Niemers, A. Widding, P. Andrews, H.-P. Schulz und H. Thomas, Arzneim. Forsch./ Drug Res. 34 (1984) 531 - 542).

Amidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließende Aminolyse hergestellt werden (G. Wagner, P. Richter und Ch. Garbe, Pharmazie 29 (1974) 12 - 15). Eine weitere Methode, Amidine herzustellen, ist die Anlagerung von $H_2S$ an die Cyanogruppe, gefolgt von einer Methylierung des entstandenen Thioamids und anschließender Umsetzung mit Ammoniak (DDR-Patent Nr. 235 866).

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon, neben üblichen pharmazeutisch einwandfreien Träger- und Zusatzstoffen enthalten. Die Zubereitungen enthalten normalerweise etwa 0,5 bis 90 Gew. % der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z. B. in Form von Suppositorien oder parenteral, z. B. in Form von Injektions- oder Infusionslösungen oder Mikrokapseln, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln oder Implantate eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihre pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüber hinaus lassen sich die Verbindungen beispielsweise auch mit nootrop wirksamen Substanzen, wie z. B. Piracetam, oder ZNS-aktiven Substanzen, wie Pirlindol, Sulpirid etc. kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt

die Tagesdosis im allgemeinen etwa 0,01 bis 0,3 mg/kg, vorzugsweise 0,05 bis 0,1 mg/kg Körpergewicht.

Die Tagesdosis kann, insbesondere bei der Applikation größerer Mengen, in mehrere, z. B. 2, 3, oder 4, Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer pharmazeutisch akzeptablen Salze pro Dosis.

Die erfindungsgemäßen Harnstoffderivate der Formel I haben die Fähigkeit, die Bindung von Fibrinogen, Fibronectin und des von Willebrand-Faktors an Integrinrezeptoren zu hemmen. Sie beeinflussen auf diese Weise die Zell-Zell- und Zell-Matrix-Wechselwirkung und können so die Bildung von Blutplättchenthromben verhindern. Integrine sind Zellmembran-Glykoproteine und vermitteln Zelladhäsion durch Wechselwirkung mit einer Vielzahl extrazellulärer Proteine wie Fibronectin, Laminin, Kollagen, Vitronectin und von Willebrand-Faktor oder mit anderen Zellmembranproteinen wie z. B. ICAM-1. Ein wichtiger Rezeptor aus der Integrinfamilie ist das auf Blutplättchen lokalisierte Glykoprotein IIb/IIIa (Fibrinogen-Rezeptor) - ein Schlüsselprotein der Plättchen-Plättchen-Wechselwirkung und Thrombenbildung. Ein zentrales Fragment in der Rezeptorerkennungssequenz dieser Proteine ist das Tripeptid Arg-Gly-Asp (E. Ruoslahti und M.D. Pierschbacher, Science 238 (1987) 491 - 497; D.R. Phillips, I.F. Charo, L.V. Parise und L.A. Fitzgerald, Blood 71 (1988) 831 - 843).

Eine Anwendung finden die Harnstoffderivate der allgemeinen Formel I daher zur Prophylaxe und Therapie von arteriellen Gefäßerkrankungen wie akutem Myokardinfarkt in Kombination mit Lysetherapie, post-Infarktbehandlung, Sekundärprävention des Myokardinfarktes, Reocclusionsprophylaxe nach Lyse und Dilatation, instabiler Angina Pectoris, transitorischer ischämischer Attacken, Schlaganfall, koronarer Bypass-Operation und Reocclusionsprophylaxe des Bypasses, Lungenembolie, peripherer arterieller Verschlußkrankheiten, dissezierendem Aneurysma, zur Therapie venöser und mikrozirkulatorischer Gefäßerkrankungen wie tiefer Venenthrombose, disseminierter intravaskuläre Gerinnung, post-operativem und post-partum Trauma, chirurgischem oder infektiösem Schock, Septicämie, zur Therapie bei Erkrankungen mit hyperreagiblen Thrombozyten, thrombotische thrombozytopenische Purpura, Preeklampsie, prämenstruelles Syndrom, Dialyse, extrakorporale Zirkulation; eine weitere Anwendung ist bei Entzündungen und bei der Behandlung von Tumoren gegeben. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen. Verwendet werden gelfiltrierte Blutplättchen aus humanem Spenderblut, die mit ADP oder Thrombin aktiviert werden.

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben wird der Ki-Wert der Bindungshemmung von $^{125}$I-Fibrinogen nach Stimulierung mit ADP (10 μM). (Literatur: J.S. Bennett u. G. Vilaire, J. Clin. Invest. 64 (1979) 1393 - 1401; E. Kornecki et al., J. Biol. Chem. 256 (1981), 5695 - 5701; G.A. Marguerie et al., J. Biol. Chem. 254 (1979) 5357 - 5363; G.A. Marguerie et al., J. Biol. Chem. 255 (1980) 154 - 161).

Bei dieser Prüfung wurde für die Verbindung des nachfolgenden Beispiels 1 folgendes Ergebnis erhalten:

| Beispiel | Ki (μM), ADP stimuliert |
|---|---|
| 1 | 0,03 |

Als funktioneller Test wird die Hemmung der Aggregation gelfiltrierter Human-Thrombozyten nach ADP- oder Thrombin-Stimulierung durch die erfindungsgemäßen Verbindungen gemessen. Angegeben ist der $IC_{50}$-Wert der Hemmung (Literatur: G.A. Marguerie et al., J. Biol. Chem. 254 (1979), 5357-5363)

Bei dieser Prüfung wurden für die Verbindungen der nachstehenden Beispiele 1, 6, 7, 8 und 10 folgende Ergebnisse erhalten:

| Beispiel | ADP-stimuliert | Thrombin-stimuliert |
|---|---|---|
| | $IC_{50}$(μM) | $IC_{50}$ (μM) |
| 1 | 0,15 | 0,1 |
| 6 | 0,75 | 0,3 |
| 7 | 3.0 | 1,5 |
| 8 | 2,0 | 2,0 |
| 10 | 8,5 | 3,0 |

**BEISPIELE**

Beispiel 1

**(3-(4-(Amino-imino-methyl)-phenyl)-ureido)-acetyl-L-aspartyl-L-phenylglycin**

a) 3-(4-Cyanophenyl)-ureido-essigsäureethylester

Zu einer gekühlten Lösung (0°C) von 3 g (23 mmol) Isocyanatoessigsäureethylester in 10 ml Dimethoxyethan wird eine Lösung von 2,74 g (23 mmol) 4-Aminobenzoesäurenitril in 10 ml Dimethoxyethan zugetropft und bei Raumtemperatur gerührt. Das Produkt wird abfiltriert und mit Dimethoxyethan gewaschen.
Ausbeute: 2,56 g (45 %)
Schmelzpunkt: 142°C

b) 3-(4-(Ethoxy-imino-methyl)-phenyl)-ureido-essigsäureethylester-Hydrochlorid

In die Suspension von 2,3 g (9,3 mmol) 3-(4-Cyanophenyl)ureido-essigsäureethylester in 50 ml wasserfreiem Ethanol wird unter Rühren und Kühlen (0°C) trockenes HCl-Gas eingeleitet. Nach 30 h wird im Vakuum eingeengt. Der Rückstand wird mit Ether verrührt und abfiltriert.
Ausbeute: 2,68 g (87 %)
Schmelzpunkt: 154-157°C.

c) 3-(4-Amino-imino-methyl)-phenyl)-ureido-essigsäureethylester-hydrochlorid

Zur Suspension von 2,68 g (8,1 mmol) 3-(4-(Ethoxy-iminomethyl)-phenyl)-ureido-essigsäureethylester-Hydrochlorid in 25 ml wasserfreiem Ethanol gibt man 5,8 ml (1,5 Äquiv.) ethanolische Ammoniaklösung. Nach 5 Tagen Rühren bei Raumtemperatur wird abfiltriert und mit Ethanol nachgewaschen.
Ausbeute: 1,66 g (68 %)

d) 3-(4-(tert.-Butyloxycarbonyl-amino-imino-methyl)phenyl)-ureido-essigsäureethylester

Eine Suspension von 1,71 g (5,7 mmol) 3-(4-Amino-iminomethyl)-phenyl)-ureido-essigsäureethylester-Hydrochlorid, 2,48 g (11,4 mmol) Di-tert.-butyl-dicarbonat und 2,86 g Natriumhydrogencarbonat in 100 ml Ethanol wird 10 h auf 50°C erhitzt. Es wird abfiltriert und das Filtrat im Vakuum eingeengt. Der Rückstand wird mit Ethylacetat versetzt und mit Wasser gewaschen. Nach dem Trocknen ($Na_2SO_4$) wird im Vakuum eingeengt.
Ausbeute: 1,64 g (79 %)
Schmelzpunkt: 167-172°C.

e) 3-(4-(tert.-Butyloxycarbonyl-amino-imino-methyl)-phenyl)-ureido-essigsäure-Natriumsalz

1,6 g (4,4 mmol) 3-(4-(tert.-Butyloxycarbonyl-amino-imino-methyl)-phenyl)-ureido-essigsäureethylester werden mit 0,18 g (4,4 mmol) NaOH in 50 ml Ethanol und 1 ml Wasser versetzt. Nach 20 h Rühren bei Raumtemperatur wird gefriergetrocknet.
Ausbeute: 1,52 g (97 %)
MS: 359 (M+1, Na-Salz), 337 (M+1, Säure).

f) 3-(4-(tert.-Butyloxycarbonyl-amino-imino-methyl)-phenyl)-ureido-acetyl-L-Asp(OtBu)-L-phenylglycin-OtBu

0,5 g (1,4 mmol) 3-(4-(tert.-Butyloxycarbonyl-amino-imino-methyl)-phenyl)-ureido-essigsäure-Natriumsalz und 0,58 g (1,4 mmol) L-Aspartyl(OtBu)-L-phenylglycin-OtBu werden in 30 ml DMF gelöst und bei 0°C unter Rühren mit 0,32 g (1,5 mmol) Dicyclohexylcarbodiimid, 0,19 g (1,4 mmol) Hydroxybenzotriazol und 2 ml Ethylmorpholin versetzt. Nach 72 h wird abfiltriert und im Vakuum eingeengt. Der Rückstand wird in Ethylacetat aufgenommen, mit gesättigter Natriumhydrogencarbonat-Lösung und Wasser gewaschen, getrocknet und am Rotationsverdampfer eingeengt.
Ausbeute: 1,02 g (100 %)

g) (3-(4-(Amino-imino-methyl)-phenyl)-ureido)-acetyl-L-aspartyl-L-phenylglycin

1,02 g (1,4 mmol) 3-(4-(tert.-Butyloxycarbonyl-amino-imino-methyl)-phenyl)-ureido-acetyl-L-Asp(OtBu)-L-phenylglycin-OtBu werden mit 1,68 g (14,6 mmol) Trifluoressigsäure (99 %), 0,11 ml Wasser und 2 ml Dichlormethan versetzt und 24 h bei Raumtemperatur belassen. Es wird im Vakuum eingeengt und über Sephadex (LH-20) chromatographiert (Butanol/Eisessig/Wasser).
Ausbeute: 120 mg (15 %)
Schmelzpunkt: 194°C (Zers.)
$[\alpha]_D^{20}$ = +27,4° (c = 0,365, Eisessig)
$^1$H-NMR ($D_6$-DMSO): δ = 7,71 (d, 2 H); 7,60 (d, 2 H); 7,40 - 7,20 (m, 5 H); 6,74 (m, 1 H); 5,08 (m, 1 H); 4,69 (m, 1H); 3,77 (m, 2 H); 2,74 - 2,60 (m, 2H); 1,89 (s, 3 H; Essigsäure).

Beispiel 2

**(3-(4-(Amino-imino-methyl)-phenyl)-ureido)-acetyl-L-aspartyl(O-isopropyl)-L-phenylglycin-isopropylester-Trifluoracetat**

a) (3-(4-(tert.-Butyloxycarbonyl-amino-imino-methyl)-phenyl)-ureido)-acetyl-L-aspartyl(O-isopropyl)-L-phenyl glycin-isopropylester

1,39 g (3,87 mmol)(3-(4-(tert.-Butyloxycarbonyl-amino-imino-methyl)phenyl)-ureido)-essigsäure-Natriumsalz und 1,50 g (3,87 mmol) L-Aspartyl(O-isopropyl)-L-phenylglycin-isopropylester werden in 30 ml DMF gelöst und bei 0°C unter Rühren mit 0,88 g (4,26 mmol) N,N'-Dicyclohexylcarbodiimid, 0,52 g (3,87 mmol) Hydroxybenzotriazol und 0,40 ml Ethylmorpholin versetzt. Nach 18 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeeingt. Der Rückstand (3,92 g) wird ohne weitere Reinigung weiter umgesetzt.

b) (3-(4-(Amino-imino-methyl)-phenyl)-ureido)-acetyl-L-aspartyl(O-isopropyl)-L-phenylglycin-isopropylester-Trifluoracetat

3,92 g (3,87 mmol)(3-(4-(tert.-Butyloxycarbonyl-amino-imino-methyl)-phenyl)-ureido)-acetyl-L-aspartyl(O-isopropyl)-L-phenylglycin-isopropylester werden mit 3 ml Trifluoressigsäure (99 %) und 0,3 ml Wasser versetzt und 28 h bei Raumtemperatur gerührt. Es wird gefriergetrocknet und der Rückstand über Kieselgel chromatographiert (Dichlormethan/Methanol 9:1, dann 8:2, dann Dichlormethan/Methanol/Eisessig 9:1:1).
Ausbeute: 730 mg (29 %)
Schmelzpunkt: 104°C bis 109°C
MS (FAB): 570,2 $[M+1]^+$
$[\alpha]_D$ = +5(c = 0,6 Methanol)
$^1$H-NMR ($D_6$-DMSO):δ = 9,20 (br.s,NH), 8,63 (d,NH), 8,31 (d,NH), 7,77 (d,2 H), 7,60 (d,2 H), 7,37 (m,5 H), 6,89 (t,NH), 5,31 (d,1 H), 4,86 (m,3 H), 3,76 (m,2 H), 2,74 (dd,1 H), 2,52 (dd,1 H), 1,26-1,03 (m,12 H).

Beispiel 3

**(3-(4-(Methoxycarbonylamino-imino-methyl)-phenyl)-ureido)-acetyl-L-aspartyl(O-methyl)-L-phenylglycinmethylester**

a) (3-(4-(Methoxycarbonyl-amino-imino-methyl)-phenyl)-ureido)-essigsäureethylester

Zur Lösung von 5 g (18,92 mmol)(3-(4-(Amino-imino-methyl)-phenyl)-ureido)-essigsäureethylester in 53 ml Triethylamin und 100 ml DMF tropft man 1,74 g (22,7 mmol) Chlorameisensäuremethylester. Nach 7 d bei Raumtemperatur wird abfiltriert und im Vakuum eingeeingt. Der Rückstand wird über Kieselgel chromatographiert (Ethylacetat/Methanol 95:5).
Ausbeute: 5,18 g (85 %).

b) (3-(4-(Methoxycarbonyl-amino-imino-methyl)-phenyl)-ureido)-essigsäure-Natriumsalz

5,15 g (15,98 mmol)(3-(4-(Methoxycarbonyl-amino-imino-methyl)-phenyl)-ureido)-essigsäureethylester werden in 200 ml Ethanol gelöst und mit 7,99 ml (15,98 mmol) einer 2N NaOH versetzt und 24 h bei Raumtemperatur gerührt. Dann wird im Vakuum eingeengt und der Rückstand gefriergetrocknet.
Ausbeute: 4,90 g (97 %).

c) (3-(4-(Methoxycarbonyl-amino-imino-methyl)-phenyl)ureido)-acetyl-L-aspartyl(O-methyl)-L-phenylglycin methylester

1,20 g (3,78 mmol)(3-(4-(Methoxycarbonyl-amino-imino-methyl)-phenyl)-ureido)-essigsäure-Natriumsalz und 1,98 g (3,78 mmol) L-Aspartyl(O-methyl)-L-phenylglycin-methylester-Trifluoracetat werden in 40 ml DMF gelöst und bei 0°C unter Rühren mit 0,86 g (4,16 mmol) N,N'-Dicyclohexylcarbodiimid, 0,51 g (3,78 mmol) Hydroxybenzotriazol und 1 ml Ethylmorpholin versetzt. Nach 20 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt und der Rückstand über Kieselgel chromatographiert (Dichlormethan/Methanol 98:2, dann 95:5).
Ausbeute: 780 mg (36 %)
MS (FAB): 571,9 $[M+1]^+$
Schmelzpunkt: 210°C bis 212°C
$[\alpha]_D$ = +49,73° (c = 0,9, Eisessig)
$^1$H-NMR ($D_6$-DMSO): δ = 9,14 (br.s,NH), 8,37 (d,NH), 7,89 (d,2 H), 7,46 (d,2 H), 7,37 (m,5 H), 6,49 (t,NH), 5,40 (d,1 H), 4,83 (m,1 H), 3,77 (m,2 H), 3,60 (s,3 H), 3,57 (s,3 H), 2,77 (dd,1 H), 260 (dd,1 H).

Beispiel 4

**(3-(4-(Benzyloxycarbonylamino-imino-methyl)-phenyl)-ureido)-acetyl-L-aspartyl(O-methyl)-L-phenylglycin-methylester**

a) (3-(4-(Benzyloxycarbonyl-amino-imino-methyl)-phenyl)-ureido)-essigsäureethylester

0,50 g (1,66 mmol)(3-(4-(Amino-imino-methyl)-phenyl)-ureido)-essigsäureethylester in 20 ml THF werden mit 2,7 ml IN NaOH versetzt und auf 0°C abgekühlt. Hierzu werden 0,29 ml (1,99 mmol) Chlorameisensäurebenzylester gegeben und 2 h bei Raumtemperatur gerührt. Der pH-Wert wird mit 1N NaOH zwischen 9 und 10 gehalten. Dann wird mit 50 ml Wasser versetzt und mit Ethylacetat extrahiert. Nach dem Trocknen ($MgSO_4$) wird das Lösungsmittel im Vakuum entfernt und der Rückstand mit Ether verrührt.
Ausbeute: 0,4 g (60 %).

b) (3-(4-Benzyloxycarbonyl-amino-imino-methyl)-phenyl)-ureido)-essigsäure-Natriumsalz

0,74 g (1,86 mmol)(3-(4-(Benzyloxycarbonyl-amino-imino-methyl)-phenyl)-ureido)-essigsäureethylester in 100 ml Ethanol werden mit 1,86 ml 1N NaOH versetzt und 6 h bei Raumtemperatur gerührt. Das Lösungsmittel wird im Vakuum entfernt und der Rückstand gefriergetrocknet.
Ausbeute: 0,73 g (99 %).

c) (3-(4-(Benzyloxycarbonylamino-imino-methyl)-phenyl)-ureido)-acetyl-L-aspartyl(O-methyl)-L-phenylglycin methylester

0,73 g (1,86 mmol)(3-(4-(Benzyloxycarbonyl-amino-imino-methyl)-phenyl)-ureido)-essigsäure-Natriumsalz und 0,61 g (1,86 mmol) L-Aspartyl(O-methyl)-L-phenylglycin-methylester-Trifluoracetat werden in 40 ml DMF gelöst und bei 0°C unter Rühren mit 0,42 g (2,04 mmol) N,N'-Dicyclohexylcarbodiimid und 0,25 g (1,86 mmol) Hydroxybenzotriazol versetzt. Nach 16 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt und der Rückstand über Kieselgel chromatographiert (Dichlormethan/Methanol 9:1).
Ausbeute: 304 mg (25 %)
MS (FAB): 647,8 $[M+1]^+$
Schmelzpunkt: 164°C bis 167°C
$[\alpha]_D$ = +38,71 (c = 0,62; Eisessig)
$^1$H-NMR ($D_6$-DMSO): δ = 9,17 (s,NH), 9,00 (br.s,2 NH), 8,79 (d,HN), 8,39 (d,NH), 7,93 (d,2 H), 7,50 (d,2 H), 7,39 (m, 10 H), 6,53 (t,NH), 5,44 (d,1 H), 5,11 (s,1 H), 4,83 (m,1 H), 3,81 (d,2 H), 3,64 (s,3 H), 3,61 (s,3 H), 2,81 (dd,1 H), 2,64 (dd,1 H).

Beispiel 5

**(3-(4-(Amino-imino-methyl)-phenyl)-ureido)-acetyl-L-aspartyl(O-methyl)-L-phenylglycin-methylester-Trifluoracetat**

a) (3-(4-tert.-Butyloxycarbonyl-amino-imino-methyl)-phenyl)-ureido)-acetyl-L-aspartyl(O-methyl)-L-phenylgly cin-methylester

1,92 g (5,34 mmol) (3-(4-(tert.-Butyloxycarbonyl-amino-imino-methyl)-phenyl)-ureido)-essigsäure-Natriumsalz werden in 30 ml DMF gelöst und bei 0°C unter Rühren mit 1,21 g (5,87 mmol) N,N'-Dicyclohexylcarbodiimid, 0,72 g (5,34 mmol) Hydroxybenzotriazol und 0,68 ml Ethylmorpholin versetzt. Dann werden 2,18 g (5,34 mmol) L-Aspartyl-(O-methyl)-L-phenylglycin-methylester-Trifluoracetat in 10 ml DMF gelöst und zugegeben. Nach 24 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt.
Ausbeute: 2,10 g (64 %).

b) (3-(4-(Amino-imino-methyl)-phenyl)-ureido)-acetyl-L-aspartyl(O-methyl)-L-phenylglycin-methylester-Trifluoracetat

2,10 g (3,43 mmol)(3-(4-(tert.-Butyloxycarbonyl-amino-imino-methyl)-phenyl)-ureido)-acetyl-L-aspartyl (O-methyl)-L-phenylglycin-methylester werden mit 3,95 g (34,3 mmol) Trifluoressigsäure (99 %) und 0,27 ml Wasser versetzt und 16 h bei Raumtemperatur gerührt. Dann wird im Vakuum eingeengt und der Rückstand über Kieselgel chromatographiert (Dichlormethan/Methanol/Eisessig 9:1:1).
Ausbeute: 1,19 g (55 %)
MS (FAB): 513,8 $[M+1]^+$
Schmelzpunkt: >140°C
$[\alpha]_D$ = +9,48° (c = 1,06, Methanol)
$^1$H-NMR ($D_6$-DMSO): δ = 9,50 (s,NH), 9,06 (br.s,4 NH), 8,77 (d,NH), 8,37 (d,NH), 7,77 (d,2 H), 7,60 (d,2 H), 7,37 (m, 5 H), 6,74 (t,NH), 5,43 (d,1 H), 4,80 (m,1 H), 3,79 (m,2 H), 3,63 (s,3 H), 3,60 (s,3 H), 2,80 (dd,1 H), 2,63 (dd,1 H).

Beispiel 6

**(3-(4-(Amino-imino-methyl)-phenyl)-ureido)-acetyl-L-aspartyl(O-methyl)-L-phenylglycin-methylester-Trifluoracetat**

a) (3-(4-(tert.-Butyloxycarbonyl-amino-imino-methyl)-phenyl)-ureido)-acetyl-L-aspartyl(O-tert.butyl)-L-phenyl glycin-methylester

1,00 g (2,78 mmol)(3-(4-(tert.-Butyloxycarbonyl-amino-imino-methyl)-phenyl)-ureido)-essigsäure-Natriumsalzwerden in 30 ml DMF gelöst und bei 0°C unter Rühren mit 0,63 g (3,06 mmol) N,N'-Dicyclohexylcarbodiimid und 0,38 g (2,78 mmol) Hydroxybenzotriazol versetzt. Dann werden 1,04 g (2,78 mmol) L-Aspartyl(O-tert.butyl)-L-phenylglycin-methylester-Hydrochlorid in 10 ml DMF gelöst und zugegeben. Nach 18 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt.
Ausbeute: 2,01 g (100 %, enthält noch N,N'-Dicyclohexylharnstoff)

b) (3-(4-(Amino-imino-methyl)-phenyl)-ureido)-acetyl-L-aspartyl-L-phenylglycin-methylester-Trifluoracetat

2,01 g (2,78 mmol)(3-(4-(tert.-Butyloxycarbonyl-amino-imino-methyl)-phenyl)-ureido)-acetyl-L-aspartyl(O-tert.-butyl)-L-phenylglycin-methylester werden in 2,16 ml Trifluoressigsäure (99 %) und 0,22 ml Wasser gelöst und 5 h bei Raumtemperatur gerührt. Das Lösungsmittel wird im Vakuum entfernt und der Rückstand über Sephadex (LH-20) chromatographiert (Butanol/Wasser/Eisessig).
Ausbeute: 713 mg (59 %)
MS (FAB): 499,8 $[M+1]^+$
Schmelzpunkt: >220°C (Zers.)
$[\alpha]_D$ = +19,35° (c = 0,16, Methanol)
$^1$H-NMR ($D_6$-DMSO): δ = 9,09 (br.s,2 NH), 8,89 (br.s,2 NH, 8,69 (d,NH), 8,36 (d,NH), 7,74 (d,2 H), 7,57 (d,2 H), 7,37 (m,5 H), 6,66 (t,NH), 5,40 (d,1 H), 4,74 (m,1 H), 3,77 (m,2 H), 3,66 (s,3 H), 2,71 (dd,1 H), 2,53 (dd,1 H).

Beispiel 7

**3-((3-(4-(Amino-imino-methyl)-phenyl)-ureido)-acetyl-amino)-3-phenyl-propionsäure**

a) 3-((3-(4-(tert.-Butyloxycarbonyl-amino-imino-methyl)-phenyl)-ureido)-acetylamino)-3-phenyl-propionsäurebenzyl ester

0,20 g (0,55 mmol) (3-(4-(tert.-Butyloxycarbonyl-amino-iminomethyl-phenyl)-ureido)-essigsäure-Natriumsalz werden in 20 ml DMF gelöst und bei 0°C unter Rühren mit 0,12 g (0,60 mmol) N,N'-Dicyclohexylcarbodiimid und 0,07 g (0,55 mmol) Hydroxybenzotriazol versetzt. Dann werden 0,14 g (0,55 mmol) 3-Amino-3-phenylpropionsäurebenzylester in 5 ml DMF gelöst und zugegeben. Nach 24 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt. Der Rückstand (0,39 g) wird ohne weitere Reinigung umgesetzt.

b) 3-((3-(4-(Amino-imino-methyl)-phenyl)-ureido)-acetyl-amino)-3-phenylpropionsäure

0,38 g (0,66 mmol) 3-((3-(4-(tert.-Butyloxycarbonylamino-imino-methyl)-phenyl)-ureido)-acetylamino)-3-phenylpropionsäurebenzylester werden in 30 ml DMF gelöst und mit 0,07 g Pd/C (10 %) versetzt. Dann wird-24 h Wasserstoff durchgeleitet, abfiltriert, im Vakuum eingeengt und der Rückstand mit 1,53 ml Trifluoressigsäure (99 %) und 0,15 ml Wasser versetzt. Nach 48 h Rühren bei Raumtemperatur wird im Vakuum eingeengt und der Rückstand über Sephadex (LH-20) chromatographiert.
Ausbeute: 28 mg (11 %)
MS (FAB): 384,8 $[M+1]^+$
Schmelzpunkt: >134°C (Zers.)
$^1$H-NMR ($D_6$-DMSO): $\delta$ = 9,20-8,91 (br.s,4 NH) 8,60 (d,NH), 7,71 (d,2 H), 7,57 (d,2 H), 7,31 (m,5 H), 6,63 (t,NH), 5,20 (m,1 H), 3,74 (m,2 H), 2,66 (m,2 H).

Beispiel 8

**(3-(4-(Amino-imino-methyl)-phenyl)-ureido)-acetyl-L-aspartyl-benzylamid**

a) (3-(4-(tert.Butyloxycarbonyl-amino-imino-methyl)-phenyl)-ureido)-acetyl-L-aspartyl(O-tert.butyl)-benzylamid

0,50 g (1,39 mmol) (3-(4-(tert.Butyloxycarbonyl-amino-imino-methyl)-phenyl)-ureido)-essigsäure Natriumsalz werden in 25 ml DMF gelöst und bei 0°C unter Rühren mit 0,32 g (1,53 mmol) N,N'-Dicyclohexylcarbodiimid und 0,19 g (1,39 mmol) Hydroxybenzotriazol versetzt. Dann werden 0,54 g (1,39 mmol) L-Aspartyl(O-tert.butyl)-benzylamid-Hydrochlorid in 5 ml DMF gelöst und zugegeben. Nach 16 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt.
Ausbeute: 890 mg (100 %, enthält noch N,N'-Dicyclohexylharnstoff)

b) (3-(4-(Amino-imino-methyl)-phenyl)-ureido)-acetyl-L-aspartyl-benzylamid

0,89 g (1,39 mmol)(3-(4-(tert.-Butyloxycarbonyl-amino-imino-methyl)-phenyl)-ureido)-acetyl-L-asparty(O-tert.-butyl)-benzylamid werden mit 2,14 ml Trifluoressigsäure (99 %) und 0,32 ml Wasser versetzt und 22 h bei Raumtemperatur gerührt. Dann wird im Vakuum eingeengt und der Rückstand über Sephadex (LH-20) chromatographiert.
Ausbeute: 440 mg (72 %)
MS (FAB): 441,6 $[M+1]^+$
Schmelzpunkt: 209°C bis 211°C (Zers.)
$[\alpha]_D$ = -29,57° (c = 0,58, DMF)
$^1$H-NMR ($D_6$-DMSO): $\delta$ = 9,00 (m,4 NH), 8,40 (m,2 NH), 7,71 (d,2 H), 7,57 (d,2 H), 7,57 (d,2 H), 7,27 (m,5 H), 6,71 (t, NH), 4,29 (d,2 H), 3,80 (m,2 H), 2,70 (dd,1 H), 2,56 (dd,1 H).

Beispiel 9

**a) 3-(3-(3-(3-Amino-propyl)-ureido)-benzoylamino)-propionsäure**

a) 3-(3-(3-tert.-Butyloxycarbonylamino-propyl)-ureido)benzoesäureethylester

2,00 g (8,12 mmol) 3-Isocyanatobenzoesäureethylester (78 %) werden in 10 ml DMF gelöst. Bei 2-8°C wird eine Lösung von 1,42 g (8,12 mmol) 1-Amino-3-tert.-butyloxy-carbonylamino-propan in 20 ml DMF langsam zugetropft. Nach 2 h Rühren bei Raumtemperatur wird das Lösungsmittel im Vakuum entfernt, der Rückstand mit Ethylacetat versetzt und mit Wasser gewaschen. Nach dem Trocknen ($MgSO_4$) wird abfiltriert und das Lösungsmittel im Vakuum entfernt. Der Rückstand wird über Kieselgel chromatographiert.
Ausbeute: 2,55 g (86 %).

b) 3-(3-(3-tert.-Butyloxycarbonylamino-propyl)-ureido)benzoesäure

2,50 g (6,84 mmol) 3-(3-(3-tert.-Butyloxycarbonylaminopropyl)-ureido)-benzoesäureethylester werden in 80 ml Ethanol gelöst und mit 3,42 ml 2N NaOH versetzt. Nach 7 d bei Raumtemperatur wird im Vakuum eingeengt. Der Rückstand wird mit Wasser versetzt, mit Citronensäure angesäuert und mit Ethylacetat extrahiert. Nach dem Trocknen ($MgSO_4$) wird abfiltriert und das Lösungsmittel im Vakuum entfernt.

c) 3-(3-(3-(3-tert.-Butyloxycarbonylamino-propyl)-ureido)-benzoylamino)-propionsäureethylester

0,45 g (1,33 mmol) 3-(3-(3-tert.-Butylcarbonylaminopropyl)-ureido)-benzoesäure werden in 20 ml DMF gelöst und bei 0°C unter Rühren mit 0,30 g (1,47 mmol) N,N'-Dicyclohexylcarbodiimid und 0,18 g (1,33 mmol) Hydroxybenzotriazol versetzt. Dann werden 0,21 g (1,33 mmol) β-Alaninethylester-Hydrochlorid und 0,51 ml N-Ethyl-morpholin in 5 ml DMF gelöst und zugegeben. Nach 20 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt.
Ausbeute: 0,65 g (100 %, enthält noch N,N'-Dicyclohexylharnstoff)

d) 3-(3-(3-(3-tert.-Butyloxycarbonylamino-propyl)-ureido)-benzoylamino)-propionsäure

0,61 g (1,33 mmol) 3-(3-(3-(3-tert.-Butyloxycarbonylamino-propyl)-ureido)-benzoylamino)-propionsäureethylester werden in 50 ml Ethanol gelöst und mit 0,73 ml 2N NaOH versetzt. Nach 25 h bei Raumtemperatur wird das Lösungsmittel im Vakuum entfernt, der Rückstand mit 20 ml Wasser und 20 ml Ethylacetat versetzt und mit Citronensäure angesäuert. Die Phasen werden getrennt und die wäßrige Phase wird noch zweimal mit je 20 ml Ethylacetat extrahiert. Nach dem Trocknen ($MgSO_4$) wird das Lösungsmittel im Vakuum entfernt und der Rückstand (0,62 g) ohne weitere Reinigung eingesetzt.

e) 3-(3-(3-(3-Amino-propyl)-ureido)-benzoylamino)-propionsäure

0,62 g (1,33 mmol) 3-(3-(3-(3-tert.-Butyloxycarbonylamino-propyl)-ureido)-benzoylamino)-propionsäure werden mit 2 ml Trifluoressigsäure und 0,30 ml Wasser versetzt und 19 h bei Raumtemperatur gerührt. Das Lösungsmittel wird im Vakuum entfernt und der Rückstand über Sephadex (Butanol/Wasser/Eisessig) chromatographiert.
Ausbeute: 380 mg (93 %)
MS (FAB): 309,8 $[M+1]^+$
Schmelzpunkt: Öl
$^1$H-NMR ($D_6$-DMSO, $CF_3COOH$): $\delta$ = 7,83 (m,1 H), 7,57 (m,1 H), 7,33 (m,2 H), 3,46 (m,2 H), 3,19 (m,2 H), 2,83 (m,2 H), 1,71 (m,2 H).

Beispiel 10

**3-(3-(3-Amino-propyl)-ureido)-benzoyl-L-aspartyl-L-phenylglycin**

a) 3-(3-(3-tert.-Butyloxycarbonylamino-propyl)-ureido)-benzoyl-L-aspartyl(O-tert.-butyl)-L-phenylglycin-tert.-butylester

0,90 g (2,67 mmol) 3-(3-(3-tert.-Butloxycarbonylaminopropyl)-ureido)-benzoesäure werden in 15 ml DMF gelöst

und bei 0°C unter Rühren mit 0,61 g (2,93 mmol) N,N'-Dicyclohexylcarbodiimid und 0,36 g (2,67 mmol) Hydroxybenzotriazol versetzt. Dann werden 1,11 g (2,67 mmol) L-Aspartyl(O-tert.butyl)-L-phenylglycin-tert.-butylester-Hydrochlorid und 0,34 ml N-Ethylmorpholin in 5 ml DMF gelöst und zugegeben. Nach 17 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt.
Ausbeute: 1,82 g (98 %).

b) 3-(3-(3-Amino-propyl)-ureido)-benzoyl-L-aspartyl-L-phenylglycin

1,82 g (2,61 mmol) 3-(3-(3-tert.-Butyloxycarbonylaminopropyl)-ureido)-benzoyl-L-aspartyl(O-tert.-butyl)-L-phenylglycin-tert.-butylester werden mit 4 ml Trifluoressigsäure (99 %) und 0,4 ml Wasser versetzt und 17 h bei Raumtemperatur gerührt. Das Lösungsmittel wird im Vakuum entfernt und der Rückstand über Sephadex (Butanol/Wasser/Eisessig) chromatographiert.
Ausbeute: 1,04 g (82 %)
MS (FAB): 487,2 $[M+1]^+$
Schmelzpunkt: >58°C
$[\alpha]_D$ = +27,12° (c = 1,18, Methanol)
$^1$H-NMR ($D_6$-DMSO): $\delta$ = 9,83 (s,OH), 9,61 (d,NH), 8,86 (d,NH), 7,86 (s,OH), 7,71 (br.s,3 NH), 7,60 (d,NH), 7,34 (m, 9 H), 6,46 (t,NH), 5,29 (d,1 H), 4,86 (m,1 H), 3,17 (m,2 H), 2,80 (m,4 H), 1,71 (m,2 H).

Beispiel 11

**3-(4-(Amino-imino-methyl)-benzyl)-3-benzyloxy-ureido)-acetyl-L-aspartyl-L-phenylglycin**

a) 4-(Benzyloxyimino-methyl)-benzonitril

3,07 g (23,4 mmol) 4-Cyanobenzaldehyd werden in 150 ml Ethanol gelöst, mit 3,74 g (23,4 mmol) o-Benzylhydroxylamin-Hydrochlorid und 11,7 ml 2N NaOH versetzt und 4 h zum Rückfluß erhitzt. Das Lösungsmittel wird im Vakuum entfernt, der Rückstand mit Wasser versetzt und mit Ethylacetat extrahiert. Nach dem Trocknen ($MgSO_4$) wird eingeengt und der Rückstand aus Ethanol umkristallisiert. Ausbeute: 3,48 g (63 %)
Schmelzpunkt: 95°C

b) 4-(Benzyloxyamino-methyl)-benzonitril

3,44 g (15 mmol) 4-(Benzyloximino-methyl)-benzonitril in 120 ml Ethanol werden auf -15°C abgekühlt. Dann werden 4,46 g (48 mmol, 4,85 ml) Boran-Pyridin-Komplex innerhalb von 10 Minuten und anschließend 26 ml ethanolische HCl-Lösung innerhalb von 20 Minuten zugetropft. Nach 24 h Rühren bei Raumtemperatur wird der entstandene Feststoff abfiltriert, in Dichlormethan gelöst und mit Wasser und Natriumhydrogencarbonat-Lösung gewaschen. Die organische Phase wird getrocknet und im Vakuum eingeengt.
Ausbeute: 2,73 g (76 %).

c) (3-(4-Cyano-benzyl)-3-benzyloxy-ureido)-essigsäureethylester

2,73 g (11 mmol) 4-(Benzyloxyamino-methyl)-benzonitril werden in 75 ml DMF gelöst und bei 0°C zu einer Lösung von 1,48 g Isocyanatoessigsäureethylester in 75 ml DMF zugetropft. Es wird 3 d auf 50°C erhitzt, dann im Vakuum eingeengt. Der Rückstand wird in Ethylacetat aufgenommen und mit Wasser gewaschen. Die organische Phase wird getrocknet ($MgSO_4$) und im Vakuum eingeengt. Der Rückstand wird in wenig Ethylacetat gelöst und mit Ether/Petrolether ausgefällt. Der angefallene Niederschlag wird ohne weitere Reinigung umgesetzt.
Ausbeute: 2,99 g
Schmelzpunkt: 82-84°C.

d) (3-(4-(Ethoxy-imino-methyl)-benzyl)-3-benzyloxyureido)-essigsäureethylester

2,99 g (8,14 mmol) (3-(4-Cyano-benzyl)-3-benzyloxyureido)-essigsäureethylester werden in 250 ml Ethanol gelöst und auf -30°C abgekühlt. In die Lösung wird trockenes HCL-gas eingeleitet. Nach 24 h wird überschüssiges HCL-Gas durch Stickstoff ausgetrieben und das Lösungsmittel im Vakuum entfernt. Der Rückstand kristallisiert aus Ethylacetat/Ether.
Ausbeute: 2,83 g (84 %)

Schmelzpunkt: 120-122°C.

e) (3-(4-(Amino-imino-methyl)-benzyl)-3-benzyloxyureido)-essigsäureethylester

2,83 g (6,84 mmol) (3-(4-(Ethoxy-imino-methyl)-benzyl)-3-benzyloxy-ureido)-essigsäueethylester werden in 100 ml Ethanol gelöst und mit 6,74 ml (6,84 mmol) ethanolischer Ammoniak-Lösung versetzt. Nach 24 h bei Raumtemperatur wird das Lösungsmittel im Vakuum entfernt und der Rückstand aus Ethylacetat/Ether auskristallisiert.
Ausbeute: 2,34 g (89 %)
Schmelzpunkt: 205-208°C.

f) (3-(4-(tert.-Butyloxycarbonylamino-imino-methyl)benzyl)-3-benzyloxy-ureido)-essigsäureethylester

2,30 g (5,98 mmol) (3-(4-(Amino-imino-methyl)-benzyl)-3-benzyloxy-ureido)-essigsäureethylester in 200 ml Ethanol werden mit 2,61 g (11,96 mmol) Di-tert.-butyldicarbonat und 1,50 g (17,94 mmol) Natriumhydrogencarbonat versetzt. Nach 2 d bei 50°C wird abfiltriert und im Vakuum eingeengt.
Ausbeute: 2,79 g (96 %)
Schmelzpunkt: 86-90°C.

g) (3-(4-(tert.-Butyloxycarbonylamino-imino-methyl)-benzyl)-3-benzyloxy-ureido)-essigsäure-Natriumsalz

2,78 g (5,74 mmol) (3-(4-(tert.-Butyloxycarbonylamino-imino-methyl)-benzyl)-3-benzyloxy-ureido)-essigsäureethylester in 50 ml Ethanol werden mit 0,23 g (5,74 mmol) NaOH und 1,2 ml Wasser versetzt und 18 h bei Raumtemperatur gerührt. Das Lösungsmittel wird im Vakuum entfernt und der Rückstand gefriergetrocknet.
Ausbeute: 2,6 g (94 %)
Schmelzpunkt: 129-137°C.

h) (3-(4-(tert.-Butyloxycarbonylamino-imino-methyl)-3-benzyloxy-ureido)-acetyl-L-aspartyl(O-tert.-butyl)-L-phenylglycin-tert.-butylester

0,5 g (1,04 mmol) (3-(4-(tert.-Butyloxycarbonylamino-imino-methyl)-benzyl)-3-benzyloxy-ureido)-essigsäure-Natriumsalz werden in 20 ml DMF gelöst und bei 0°C unter Rühren mit 0,24 g (1,15 mmol) N,N'-Dicyclohexylcarbodiimid und 0,14 g (1,04 mmol) Hydroxybenzotriazol versetzt. Dann werden 0,43 g (1,04 mmol) L-Aspartyl(O-tert.-butyl)-L-phenylglycin-tert.-butylester-Hydrochlorid zugegeben. Nach 19 h wird abfiltriert und im Vakuum eingeengt. Der Rückstand wird in Ethylacetat aufgenommen und mit gesättigter Natriumhydrogencarbonat-Lösung und Wasser gewaschen. Nach dem Trocknen ($MgSO_4$) wird im Vakuum eingeengt und das Produkt ohne weitere Reinigung umgesetzt.
Ausbeute: 0,93 g (enthält noch Dicyclohexylharnstoff).

i) (3-(4-(Amino-imino-methyl)-benzyl)-3-benzyloxy-ureido)-acetyl-L-aspartyl-L-phenylglycin

0,93 g (1.04 mmol) (3-(4-(tert.-Butyloxycarbonylaminoimino-methyl)-benzyl)-3-benzyloxy-ureido)-acetyl-L-aspartyl(O-tert.-butyl)-L-phenylglycin-tert.-butylester werden mit 1 ml Trifluoressigsäure (99 %) und 0,1 ml Wasser versetzt und 24 h bei Raumtemperatur gerührt. Das Lösungsmittel wird im Vakuum entfernt und der Rückstand über Sephadex (Butanol/Wasser/Eisessig) chromatographiert.
Ausbeute: 0,184 g (29 %)
MS: 605 $[M+1]^+$
Schmelzpunkt: 203-210°C
$^1$H-NMR ($D_6$-DMSO): $\delta$ = 9,80 (s,2 NH), 9,40 (s,2 NH), 8,40 (d,NH), 8,10 (d,NH), 7,74 (d,2 H), 7,49 (d,2 H), 7,43-7,00 (m,10 H), 4,86 (m,4 H), 4,67 (m,1 H), 4,54 (d,1 H), 3,89 (dd,1 H), 3,63 (dd,1 H), 2,69 (dd,1 H), 2,46 (dd,1 H).

Analog können erhalten werden:

Beispiel 12:

(3-(4-(Amino-imino-methyl)-benzyl)-ureido)-acetyl-L-aspartyl-L-phenylglycin

Beispiel 13:

(3-(4-(Amino-imino-methyl)-phenyl)-thioureido)-acetyl-L-aspartyl-L-phenylglycin

Beispiel 14:

(3-(4-(Amino-imino-methyl)-benzyl)-3-hydroxy-ureido)-acetyl-L-aspartyl-L-phenylalanin

Beispiel 15:

3-(3-(2-Guanidino-ethyl)-ureido)-benzoyl-L-aspartyl-L-valin

Beispiel 16:

1-(4-(Amino-imino-methyl)-phenylcarbamoyl)-pyrrolidin-2-yl-carbonyl-L-aspartyl-L-phenylglycin

Beispiel 17:

4-(3-(2-Amino-2-imino-ethyl)-ureido)-benzoyl-L-aspartyl-L-phenylglycin

Beispiel 18:

(3-(4-Guanidino-phenyl)-ureido)-acetyl-L-aspartyl-L-phenylglycin

Beispiel 19:

2-(3-(4-(Amino-imino-methyl)-phenyl)-ureido)-propionyl-L-aspartyl-L-phenylglycin

Beispiel 20:

3-(3-(4-(Amino-imino-methyl)-phenyl)-ureido)-propionyl-L-aspartyl-L-phenylglycin

Beispiel 21:

((3-(4-(Amino-imino-methyl)-phenyl)-ureido)-acetyl-L-aspartylamino-phenyl-methylsulfonyl)-harnstoff

Beispiel 22:

(3-(4-(Amino-imino-methyl)-phenyl)-3-methyl-ureido)-acetyl-L-aspartyl-L-phenylglycin

Beispiel 23:

(3-(4-(Amino-imino-methyl)-phenyl)-1,3-dimethyl-ureido)-acetyl-L-aspartyl-L-phenylglycin

Beispiel 24:

(3-(4-(Guanidino-phenyl)-ureido)-acetyl-L-aspartyl-L-phenylglycin

Beispiel 25:

3-(3-(4-(Amino-imino-methyl)-phenyl)-ureido)-acetyl-amino)-3-(oxazol-2-yl)-propionsäure

Beispiel 26

3-(3-(4-(Amino-imino-methyl)-phenyl)-ureido)-acetyl-amino)-pent-4-in-säure

Beispiel 27

(3-(4-(Amino-imino-methyl)-phenyl)-3-benzyl-ureido)-acetyl-L-aspartyl-L-phenylglycin

Beispiel 28

(3-(4-(((1-Acetoxy-ethoxy)-carbonyl)amino-imino-methyl)-phenyl)-ureido)-acetyl-L-aspartyl(O-methyl)-L-phenylglycin-methylester

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe | q. s. |
| Wasser (entmineralisiert oder destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |

(fortgesetzt)

| | |
|---|---|
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff . | 1,0 mg |
| Polyethylenglykol 400 | 0,3 mg |
| Natriumchlorid | 2,7 mg |
| Wasser zu Injektionszwecken auf | 1 ml |

## Patentansprüche

**1.** Harnstoffderivate der allgemeinen Formel I,

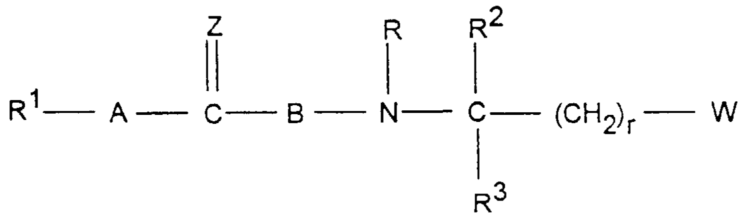

worin

| | |
|---|---|
| r | für die Zahlen 0 oder 1 steht; |
| Z | Sauerstoff oder Schwefel bedeutet; |
| W | $-COW^1$ oder Tetrazolyl bedeutet; |
| $W^1$ | Hydroxy, $(C_1-C_4)$-Alkoxy, Benzyloxy, Amino oder Mono- oder Di-$((C_1-C_8)$-alkyl)-amino bedeutet; |
| A | 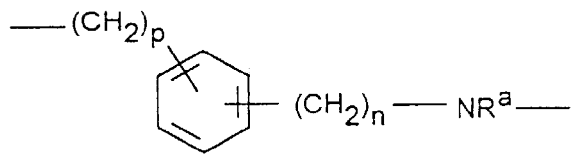 bedeutet, worin p für die Zahl 0 steht und n für die Zahlen 0 oder 1 steht; |
| B | $-NR^b-(CH_2)_m-CO-$ bedeutet, worin m für die Zahlen 1 oder 2 steht; |
| $R^a$ und $R^b$ | unabhängig voneinander Wasserstoff, Hydroxy, $(C_1-C_{18})$-Alkyl, $(C_6-C_{14})$-Aryl, $(C_6-C_{14})$-Aryl-$(C_1-C_8)$-alkyl, $(C_1-C_{28})$-Alkoxy, $(C_6-C_{14})$-Aryloxy, $(C_6-C_{14})$-Aryl-$(C_1-C_8)$-alkoxy bedeuten; |
| R | Wasserstoff oder $(C_1-C_6)$-Alkyl bedeutet; |
| $R^1$ | $-NH-X$ oder $-C(=NX)-NH_2$ bedeutet; |
| X | Wasserstoff, $(C_1-C_6)$-Alkylcarbonyl, $(C_1-C_8)$-Alkoxycarbonyl, $(C_1-C_{18})$-Alkylcarbonyloxy-$(C_1-C_6)$-alkoxycarbonyl, $(C_6-C_{14})$-Aryl-$(C_1-C_6)$-alkoxycarbonyl, Hydroxy oder einen Rest der Formel II 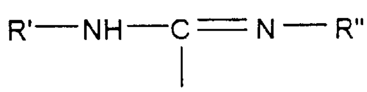 (II) bedeutet, wobei R' und R" unabhängig voneinander für Wasserstoff, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryl-$(C_1-C_6)$-alkoxycarbonyl oder Hydroxy stehen; |
| $R^2$ | Wasserstoff oder Phenyl bedeutet; |
| $R^3$ | Wasserstoff oder $-CO-NH-R^4$ bedeutet, wobei $-NH-R^4$ für den Rest einer α-Aminosäure, deren ω-Amino-$(C_2-C_8)$-alkylamid oder deren $(C_1-C_8)$-Alkyl- oder Benzylester steht, oder wobei $R^4$ Methyl bedeutet, das durch eine Aminosäureseitenkette und durch einen Rest aus der Reihe $-SO_2-OH$, $-SO_2-NHR^9$, Tetrazolyl substituiert ist; |
| $R^9$ | Wasserstoff, Aminocarbonyl, $(C_1-C_{18})$-Alkylaminocarbonyl, $(C_3-C_8)$-Cycloalkylaminocarbonyl, $(C_1-C_{18})$-Alkyl oder $(C_3-C_8)$-Cyoloalkyl bedeutet; |

sowie deren physiologisch verträgliche Salze.

**2.** Harnstoffderivate gemäß Anspruch 1, dadurch gekennzeichnet, daß in der allgemeinen Formel I

r für die Zahl 1 steht;

W -COW[1] bedeutet;
W[1] Hydroxy, ($C_1$-$C_4$)-Alkoxy, insbesondere Methoxy, Ethoxy, 2-Propyloxy, Isobutyloxy oder tert.-Butyloxy, oder Benzyloxy bedeutet;
R Wasserstoff bedeutet;
X Wasserstoff, ($C_1$-$C_6$)-Alkoxycarbonyl, ($C_1$-$C_6$)-Alkylcarbonyl, ($C_1$-$C_{18}$)-Alkylcarbonyloxy-($C_1$-$C_6$)-alkoxycarbonyl, ($C_6$-$C_{14}$)-Aryl-($C_1$-$C_6$)-alkoxycarbonyl oder einen Rest der Formel

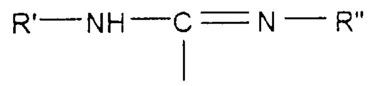

bedeutet, worin R' und R" unabhängig voneinander für Wasserstoff, ($C_1$-$C_6$)-Alkylcarbonyl, ($C_1$-$C_6$)-Alkoxycarbonyl, ($C_1$-$C_{18}$)-Alkylcarbonyloxy-($C_1$-$C_6$)-alkoxycarbonyl oder ($C_6$-$C_{14}$)-Aryl-($C_1$-$C_6$)-alkoxycarbonyl stehen;
$R^2$ Wasserstoff bedeutet;
$R^3$ -CO-NH-$R^4$ bedeutet, wobei -NH-$R^4$ für den Rest einer α-Aminosäure, deren ω-Amino-($C_2$-$C_8$)-alkylamid oder deren ($C_1$-$C_8$)-Alkyl- oder Benzylester steht, oder wobei $R^4$ Methyl bedeutet, das durch eine Aminosäureseitenkette und durch einen Rest aus der Reihe -$SO_2$-OH, -$SO_2$-NH$R^9$, Tetrazolyl substituiert ist.

**3.** Harnstoffderivate gemäß Anspruch 1 und/oder 2, dadurch gekennzeichnet, daß $R^3$ für -CO-NH-$R^4$ steht, wobei -NH-$R^4$ für den Rest der α-Aminosäuren Valin, Lysin, Phenylalanin, Phenylglycin oder 4-Chlorphenylglycin, deren ω-Amino-($C_2$-$C_8$)-alkylamide oder deren ($C_1$-$C_8$)-Alkyl- oder Benzylester steht.

**4.** Harnstoffderivate gemäß Anspruch 3, dadurch gekennzeichnet, daß der ($C_1$-$C_8$)-Alkylester der α-Aminosäuren der Methyl-, Ethyl-, Isopropyl-, Isobutyl- oder tert.-Butylester ist.

**5.** Verfahren zur Herstellung von Verbindungen der allgemeinen Formel I gemäß einem oder mehreren der Ansprüche 1 bis 4, dadurch gekennzeichnet, daß man eine Fragmentkondensation einer Verbindung der allgemeinen Formel III

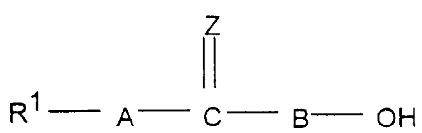

(III)

mit einer Verbindung der allgemeinen Formel IV ausführt,

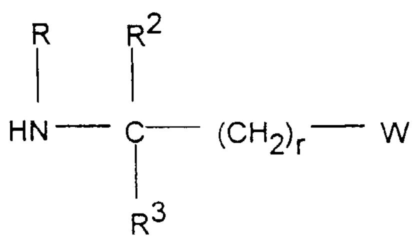

(IV)

wobei die Reste A, B, W, Z, R, $R^1$, $R^2$ und $R^3$ sowie r wie in den Ansprüchen 1 bis 4 angegeben definiert sind.

**6.** Verbindung der allgemeinen Formel I gemäß einem oder mehreren der Ansprüche 1 bis 4 und/oder ihre physiologisch verträglichen Salze zur Anwendung als Hemmstoffe der Thrombozytenaggregation, der Metastasierung von Karzinomzellen oder der Osteoclastenbindung an die Knochenoberfläche.

**7.** Pharmazeutisches Präparat, dadurch gekennzeichnet, daß es eine oder mehrere Verbindungen der allgemeinen

Formel I gemäß einem oder mehreren der Ansprüche 1 bis 4 und/oder ein oder mehrere physiologisch verträgliche Salze davon als Wirkstoff zusammen mit pharmazeutisch annehmbaren Träger- und Zusatzstoffen und gegebenenfalls noch einen oder mehrere andere pharmakologische Wirkstoffe enthält.

**8.** Verfahren zur Herstellung eines pharmazeutischen Präparates, enthaltend eine oder mehrere Verbindungen der allgemeinen Formel I gemäß einem oder mehreren der Ansprüche 1 bis 4 und/oder ein oder mehrere physiologisch verträgliche Salze davon, dadurch gekennzeichnet, daß man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

**Claims**

**1.** A urea derivative of the formula I

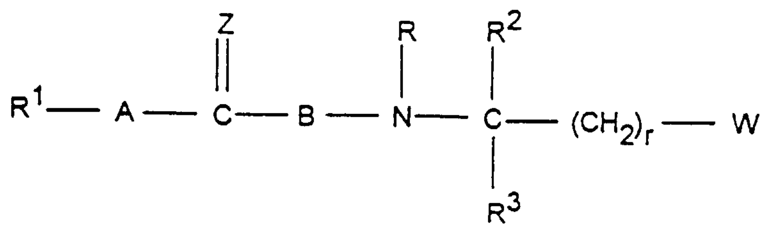

in which

| | |
|---|---|
| r | is the number 0 or 1; |
| Z | is oxygen or sulfur; |
| W | is -$COW^1$ or tetrazolyl; |
| $W^1$ | is hydroxyl, $(C_1\text{-}C_4)$-alkoxy, benzyloxy, amino or mono- or di-$((C_1\text{-}C_8)$-alkyl)amino; |
| A | is |

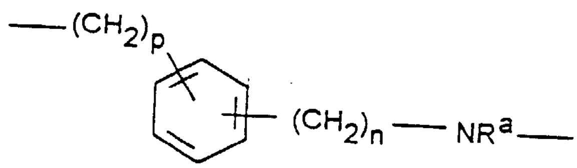

| | |
|---|---|
| | in which p is the number 0 and n is the number 0 or 1; |
| B | is -$NR^b$-$(CH_2)_m$-CO-, in which m is the number 1 or 2; |
| $R^a$ and $R^b$ | independently of one another are hydrogen, hydroxyl, $(C_1\text{-}C_{18})$-alkyl, $(C_6\text{-}C_{14})$-aryl, $(C_6\text{-}C_{14})$-aryl-$(C_1\text{-}C_8)$-alkyl, $(C_1\text{-}C_{28})$-alkoxy, $(C_6\text{-}C_{14})$-aryloxy, $(C_6\text{-}C_{14})$-aryl-$(C_1\text{-}C_8)$-alkoxy; |
| R | is hydrogen or $(C_1\text{-}C_6)$-alkyl; |
| $R^1$ | is -NH-X or -C(=NX)-$NH_2$; |
| X | is hydrogen, $(C_1\text{-}C_6)$-alkylcarbonyl, $(C_1\text{-}C_6)$-alkoxycarbonyl, $(C_1\text{-}C_{18})$-alkylcarbonyloxy-$(C_1\text{-}C_6)$-alkoxycarbonyl, $(C_6\text{-}C_{14})$-aryl-$(C_1\text{-}C_6)$-alkoxycarbonyl, hydroxyl or a radical of the formula II |

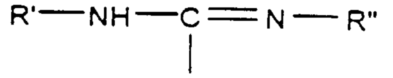
(II)

| | |
|---|---|
| | where R' and R" independently of one another are hydrogen, $(C_1\text{-}C_6)$-alkoxycarbonyl, $(C_1\text{-}C_6)$-alkylcarbonyl, $(C_1\text{-}C_{18})$-alkylcarbonyloxy-$(C_1\text{-}C_6)$-alkoxycarbonyl, $(C_6\text{-}C_{14})$-aryl-$(C_1\text{-}C_6)$-alkoxycarbonyl or hydroxyl; |
| $R^2$ | is hydrogen or phenyl; |

$R^3$ is hydrogen or -CO-NH-$R^4$, where -NH-$R^4$ is the radical of an α-amino acid, its ω-amino-($C_2$-$C_8$)-alkylamide or its ($C_1$-$C_8$)-alkyl or benzyl ester, or where $R^4$ is methyl which is substituted by an amino acid side-chain and by a radical from the group consisting of -$SO_2$-OH, -$SO_2$-NH$R^9$ and tetrazolyl;

$R^9$ is hydrogen, aminocarbonyl, ($C_1$-$C_{18}$)-alkylaminocarbonyl, ($C_3$-$C_8$)-cycloalkylaminocarbonyl, ($C_1$-$C_{18}$)-alkyl or ($C_3$-$C_8$)-cycloalkyl;

or its physiologically tolerable salts.

**2.** A urea derivative as claimed in claim 1, wherein, in the formula I

r is the number 1;

W is -CO$W^1$;

$W^1$ is hydroxyl, ($C_1$-$C_4$)-alkoxy, in particular methoxy, ethoxy, 2-propyloxy, isobutyloxy or tert-butyloxy, or benzyloxy;

R is hydrogen;

X is hydrogen, ($C_1$-$C_6$)-alkoxycarbonyl, ($C_1$-$C_6$)-alkylcarbonyl, ($C_1$-$C_{18}$)-alkylcarbonyloxy-($C_1$-$C_6$)-alkoxycarbonyl, ($C_6$-$C_{14}$)-aryl-($C_1$-$C_6$)-alkoxycarbonyl or a radical of the formula

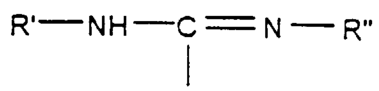,

in which R' and R" independently of one another are hydrogen, ($C_1$-$C_6$)-alkylcarbonyl, ($C_1$-$C_6$)alkoxycarbonyl, ($C_1$-$C_{18}$)-alkylcarbonyloxy-($C_1$-$C_6$)-alkoxycarbonyl or ($C_6$-$C_{14}$)-aryl-($C_1$-$C_6$)-alkoxycarbonyl;

$R^2$ is hydrogen;

$R^3$ is -CO-NH-$R^4$, where -NH-$R^4$ is the radical of an α-amino acid, its ω-amino-($C_2$-$C_8$)-alkylamide or its ($C_1$-$C_8$)-alkyl or benzyl ester, or where $R^4$ is methyl which is substituted by an amino acid side-chain and by a radical from the group consisting of -$SO_2$-OH, -$SO_2$-NH$R^9$ and tetrazolyl.

**3.** A urea derivative as claimed in claim 1 and/or 2, wherein $R^3$ is -CO-NH-$R^4$, where -NH-$R^4$ is a radical of the α-amino acids valine, lysine, phenylalanine, phenylglycine or 4-chlorophenylglycine, its ω-amino-($C_2$-$C_8$)-alkylamides or its ($C_1$-$C_8$)-alkyl or benzyl esters.

**4.** A urea derivative as claimed in claim 3, wherein the ($C_1$-$C_8$)-alkyl ester of the α-amino acids is the methyl, ethyl, isopropyl, isobutyl or tert-butyl ester.

**5.** A process for the preparation of compounds of the formula I as claimed in one or more of claims 1 to 4, which comprises carrying out a fragment condensation of a compound of the formula III

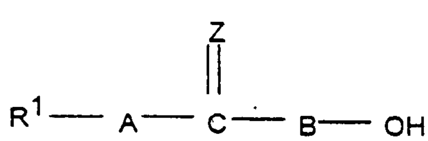

(III)

with a compound of the formula IV,

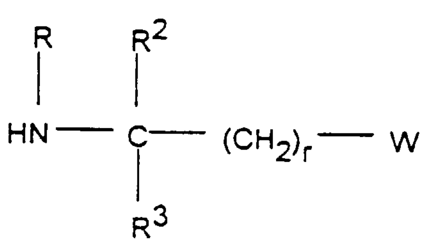

(IV)

where the radicals A, B, W, Z, R, $R^1$, $R^2$ and $R^3$, and r are defined as indicated in claims 1 to 4.

**6.** A compound of the formula I as claimed in one or more of claims 1 to 4 and/or its physiologically tolerable salts for use as inhibitors of platelet aggregation, metastasis of carcinoma cells or osteoclast binding to the bone surface.

**7.** A pharmaceutical preparation which comprises one or more compounds of the formula I as claimed in one or more of claims 1 to 4 and/or one or more physiologically tolerable salts thereof as active compound together with pharmaceutically acceptable excipients and additives and, if appropriate, additionally one or more other pharmacological active compounds.

**8.** A process for the production of a pharmaceutical preparation, comprising one or more compounds of the formula I as claimed in one or more of claims 1 to 4 and/or one or more physiologically tolerable salts thereof, which comprises bringing these into a suitable administration form together with pharmaceutically acceptable excipients and additives and, if appropriate, additionally one or more other pharmacological active compounds.

## Revendications

**1.** Dérivés d'urée de formule générale I

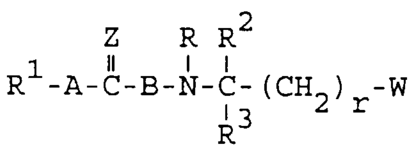

(I)

dans laquelle

| | |
|---|---|
| r | représente 0 ou 1; |
| Z | représente un atome d'oxygène ou de soufre; |
| W | représente $-COW^1$ ou le groupe tétrazolyle; |
| $W^1$ | représente un groupe hydroxyle, alcoxy en $C_1$-$C_4$, benzyloxy, amino ou mono- ou di[alkyl($C_1$-$C_8$)] amino; |
| A | représente |

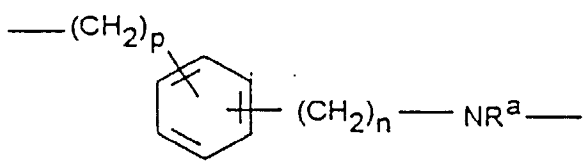

| | |
|---|---|
| | p étant égal à 0 et n représentant 0 ou 1; |
| B | représente $-NR^b-(CH_2)_m-CO-$, m représentant le nombre 1 ou 2; |
| $R^a$ et $R^b$ | représentent, indépendamment l'un de l'autre, un atome d'hydrogène ou un groupe hydroxyle, alkyle en $C_1$-$C_{18}$, aryle en $C_6$-$C_{14}$, aryl($C_6$-$C_{14}$)-alkyle($C_1$-$C_8$), alcoxy en $C_1$-$C_{28}$, aryloxy en $C_6$-$C_{14}$, aryl($C_6$-$C_{14}$)-alcoxy($C_1$-$C_8$); |
| R | représente un atome d'hydrogène ou un groupe alkyle en $C_1$-$C_6$; |
| $R^1$ | représente -NH-X ou $-C(=NX)-NH_2$; |
| X | représente un atome d'hydrogène ou un groupe alkyl-($C_1$-$C_6$)-carbonyle, alcoxy($C_1$-$C_6$)-carbonyle, alkyl-($C_1$-$C_{18}$)-carbonyloxy-alcoxy($C_1$-$C_6$)-carbonyle, aryl-($C_6$-$C_{14}$)-alcoxy($C_1$-$C_6$)carbonyle, hydroxyle ou un radical de formule II |

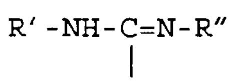

(II)

R' et R" représentant, indépendamment l'un de l'autre, un atome d'hydrogène ou un groupe alcoxy ($C_1$-$C_6$)-carbonyle, alkyl($C_1$-$C_6$)-carbonyle, alkyl($C_1$-$C_{18}$)-carbonyloxy-alcoxy($C_1$-$C_6$)-carbonyle, aryl ($C_6$-$C_{14}$)-alcoxy($C_1$-$C_6$)-carbonyle ou hydroxyle;

$R^2$ représente un atome d'hydrogène ou le groupe phényle;

$R^3$ représente un atome d'hydrogène ou un groupe -CO-NH-$R^4$, -NH-$R^4$ représentant le reste d'un α-aminoacide, d'un ω-amino-alkyl($C_2$-$C_8$)-amide de celui-ci ou d'un ester alkylique en $C_1$-$C_8$ ou benzylique de celui-ci, ou $R^4$ représentant un groupe méthyle substitué par une chaîne latérale d'aminoacide et par un radical choisi parmi les groupes -$SO_2$-OH, -$SO_2$-NH$R^9$ et tétrazolyle;

$R^9$ représente un atome d'hydrogène ou un groupe aminocarbonyle, alkyl($C_1$-$C_{18}$)aminocarbonyle, cycloalkyl-($C_3$-$C_8$)aminocarbonyle, alkyle en $C_1$-$C_{18}$ ou cycloalkyle en $C_3$-$C_8$;

ainsi que leurs sels physiologiquement acceptables.

**2.** Dérivés d'urée selon la revendication 1, caractérisés en ce que, dans la formule générale I

r représente le nombre 1;

W représente -COW$^1$;

W$^1$ représente un groupe hydroxyle, alcoxy en $C_1$-$C_4$, en particulier méthoxy, éthoxy, 2-propyloxy, isobutyloxy ou tert-butyloxy, ou benzyloxy;

R représente un atome d'hydrogène;

X représente un atome d'hydrogène ou un groupe alcoxy($C_1$-$C_6$)-carbonyle, alkyl($C_1$-$C_6$)-carbonyle, alkyl-($C_1$-$C_{18}$)-carbonyloxy-alcoxy($C_1$-$C_6$)-carbonyle, aryl($C_6$-$C_{14}$)-alcoxy($C_1$-$C_6$)-carbonyle ou un radical de formule

$$R'\text{-NH-}\underset{|}{C}\text{=N-}R''$$

dans laquelle R' et R" représentent, indépendamment l'un de l'autre, un atome d'hydrogène ou un groupe alkyl($C_1$-$C_6$)-carbonyle, alcoxy($C_1$-$C_6$)-carbonyle, alkyl($C_1$-$C_{18}$)-carbonyloxy-alcoxy($C_1$-$C_6$)carbonyle ou aryl ($C_6$-$C_{14}$)-alcoxy($C_1$-$C_6$)-carbonyle;

$R^2$ représente un atome d'hydrogène;

$R^3$ représente un groupe -CO-NH-$R^4$, dans lequel -NH-$R^4$ représente le reste d'un α-aminoacide, d'un ω-amino-alkyl($C_2$-$C_8$)amide de celui-ci ou d'un ester alkylique en $C_1$-$C_8$ ou benzylique de celui-ci ou dans lequel $R^4$ représente un groupe méthyle qui est substitué par une chaîne latérale d'aminoacide et par un radical choisi parmi les groupes -$SO_2$-OH, -SO-NH$R^9$ et tétrazolyle.

**3.** Dérivés d'urée selon la revendication 1 et/ou la revendication 2, caractérisés en ce que $R^3$ représente -CO-NH-$R^4$, -NH-$R^4$ représentant le reste des α-aminoacides valine, lysine, phénylalanine, phénylglycine ou 4-chlorophénylglycine, de leurs ω-amino-alkyl($C_2$-$C_8$)-amides ou de leurs esters alkyliques en $C_1$-$C_8$ ou benzyliques.

**4.** Dérivés d'urée selon la revendication 3, caractérisés en ce que l'ester alkylique en $C_1$-$C_8$ des α-aminoacides est l'ester méthylique, éthylique, isopropylique, isobutylique ou tert-butylique.

**5.** Procédé pour la préparation des composés de formule générale I selon une ou plusieurs des revendications 1 à 4, caractérisé en ce que l'on effectue une condensation de fragments consistant en un composé de formule générale III

$$R^1\text{-A-}\overset{Z}{\overset{\|}{C}}\text{-B-OH} \qquad \text{(III)}$$

avec un composé de formule générale IV

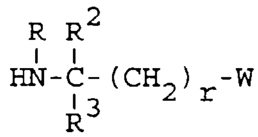

(IV)

les radicaux A, B, W, Z, R, $R^1$, $R^2$ et $R^3$ ainsi que r étant définis comme indiqué dans les revendications 1 à 4.

**6.** Composé de formule générale I selon une ou plusieurs des revendications 1 à 4 et/ou ses sels physiologiquement acceptables, pour utilisation en tant qu'inhibiteurs de l'agrégation des thrombocytes, de la production de métastases de cellules cancéreuses ou de la fixation d'ostéoclastes à la surface des os.

**7.** Composition pharmaceutique, caractérisée en ce qu'elle contient un ou plusieurs composés de formule générale I selon une ou plusieurs des revendications 1 à 4 et/ou un ou plusieurs sels physiologiquement acceptables de ceux-ci, en tant que substance active, conjointement avec des véhicules et additifs pharmaceutiquement acceptables et éventuellement encore une ou plusieurs autres substances actives pharmacologiques.

**8.** Procédé pour la préparation d'une composition pharmaceutique, contenant un ou plusieurs composés de formule générale I selon une ou plusieurs des revendications 1 à 4 et/ou un ou plusieurs sels physiologiquement acceptables de ceux-ci, caractérisé en ce qu'on les met sous une forme d'administration appropriée, conjointement avec des véhicules et additifs pharmaceutiquement acceptables et éventuellement encore une ou plusieurs autres substances actives pharmacologiques.